# EUROPÄISCHE PATENTSCHRIFT

(11) **EP 3 488 835 B1**
(45) Veröffentlichungstag und Bekanntmachung des Hinweises auf die Patenterteilung: **22.03.2023**
(21) Anmeldenummer: 18200477.0
(22) Anmeldetag: 15.10.2018
(51) Int. Cl.: A61F 9/007

(54) **VORRICHTUNG ZUM SCHNEIDEN UND ABSAUGEN VON GEWEBE AUS DEM MENSCHLICHEN ODER TIERISCHEN AUGE**
DEVICE FOR CUTTING AND ASPIRATING TISSUE FROM A HUMAN OR ANIMAL EYE
DISPOSITIF DE COUPE ET D'ASPIRATION DE TISSU DE L'OEIL HUMAIN OU ANIMAL

(30) Priorität: 24.11.2017 DE 102017221017
(43) Veröffentlichungstag der Anmeldung: 29.05.2019
(73) Patentinhaber: Geuder AG, 69126 Heidelberg (DE)
(72) Erfinder: El-Ayari, Hamadi, 60323 Frankfurt (DE); Geuder, Volker, 69126 Heidelberg (DE); Müller-Albinus, Julius, 69488 Birkenau (DE)
(74) Vertreter: Ullrich & Naumann PartG mbB

(56) Entgegenhaltungen:
- EP-A1- 2 863 853
- WO-A2-2008/079526
- DE-U1-202013 012 000
- US-A- 3 815 604
- US-A- 5 980 546

## Beschreibung

Die Erfindung betrifft eine Vorrichtung zum Schneiden und Absaugen von Gewebe aus dem menschlichen oder tierischen Auge, insbesondere zur Durchführung einer Vitrektomie oder eines Netzhautpeelings, mit einer Außenröhre und einer in der Außenröhre bewegbaren Innenröhre, wobei die Außenröhre in einem Schneidbereich mehrere seitliche Öffnungen mit zumindest jeweils einer Außenschneidkante aufweist, wobei die Innenröhre in dem Schneidbereich mehrere seitliche Öffnungen mit jeweils zumindest einer Innenschneidkante aufweist, wobei die Innenschneidkante und die Außenschneidkante beim Bewegen der Innenröhre gegenüber der Außenröhre schneidend zusammen wirken, wobei in dem Schneidbereich der Außendurchmesser der Innenröhre dem Innendurchmesser der Außenröhre entspricht oder größer als der Innendurchmesser der Außenröhre ist und dass die Wandung der Innenröhre im Schneidbereich einen Schlitz aufweist.

Grundsätzlich geht es hier um ein chirurgisches Schneidinstrument zur Entnahme von Gewebe aus dem Auge. Mit dem Instrument lässt sich das Gewebe - am bzw. im Auge - schneiden und vom Auge bzw. aus dem Auge heraus absaugen. Im Konkreten kann es sich dabei um ein ganz besonderes Schneidinstrument handeln, mit dem im Rahmen der Vitrektomie der Glaskörper im Auge zerstört bzw. zerkleinert und aus dem Auge entfernt/abgesaugt wird. Auch lassen sich mit diesem Instrument Blut, Blutgerinnsel und bindegewebsähnliche Veränderungen sowie Bereiche der Netzhaut im Rahmen eines Netzhautpeelings entfernen.

Zum Stand der Technik sei lediglich beispielhaft auf die DE 10 2010 050 337 A1 verwiesen. Aus dieser Druckschrift ist eine entsprechende Vorrichtung bekannt, wobei dort sowohl die äußere Röhre als auch die innere Röhre jeweils zwei seitliche Ausnehmungen mit Doppelschneidfunktion haben. Ähnliche Vorrichtungen sind aus der US 5,474,532 und US 5,106,364 bekannt. Des Weiteren betreffen die Dokumente WO 2008/079526 A2, DE 20 2013 012 000 U1, US 3 815 604 A, EP 2 863 853 A1 und US 5 980 546 A ähnliche chirurgische Instrumente.

Bei diesen Vorrichtungen ist typisch, dass zwischen der Innenwandung der äußeren Röhre und der Außenwandung der inneren Röhre ein äußerst geringes Spiel realisiert sein muss, so dass die jeweiligen Schneidkanten in idealer Weise zusammenwirken. Das Gewebe soll durch Zusammenwirken der Schneidkanten geschnitten und nicht etwa gequetscht oder abgeschert werden. Ein ideales Zusammenwirken der Schneidkanten bei geringstmöglichem Spiel ist angestrebt.

Bei der hier in Rede stehenden Vorrichtung zur Anwendung in der Augenchirurgie, sind kleinste Abmessungen zu realisieren. So gibt es Vorrichtungen der gattungsbildenden Art, bei denen die innere Röhre eine Wandstärke von vier Hundertstel Millimeter hat. Die äußere Röhre kann einen Außendurchmesser im Bereich von 0,5 bis 0,9 mm und einen Innendurchmesser von 0,35 bis 0,37 mm aufweisen. Entsprechend ist der Außendurchmesser der inneren Röhre, mit geringstmöglichem Spiel, an den Innendurchmesser der äußeren Röhre anzupassen. Dies ist in der Konstruktion aufwändig und verursacht daher erhebliche Kosten bei der Fertigung, jedenfalls dann, wenn ein hinreichend gutes Schneidergebnis erzielt werden soll.

In der DE 10 2013 201 784 B4 ist eine Vorrichtung offenbart, bei der die Innenröhre zumindest eine Biegung aufweist, um mit Vorspannung innerhalb der Außenröhre zu verlaufen. Hierdurch soll ein Kontakt beider Röhren über alle Schneidkanten hinweg gewährleistet werden. Diese Konstruktion ist jedoch sehr aufwändig und daher teuer in der Herstellung.

Der vorliegenden Erfindung liegt daher die Aufgabe zugrunde, eine Vorrichtung zum Schneiden und Absaugen von Gewebe aus dem menschlichen oder tierischen Körper derart auszugestalten und weiterzubilden, dass die Innenröhre mit geringstem Spiel in der Außenröhre geführt ist.

Erfindungsgemäß wird die voranstehende Aufgabe durch die Merkmale des Anspruches 1 gelöst. Danach ist die in Rede stehende Vorrichtung dadurch gekennzeichnet, dass die seitlichen Öffnungen der Außenröhre und/oder die seitlichen Öffnungen der Innenröhre wabenförmig ausgebildet sind.

In erfindungsgemäßer Weise ist zunächst erkannt worden, dass durch eine Schlitzung des Innenrohres auf verblüffend einfache Weise eine reproduzierbare Vorspannung des Innenrohres gegenüber des Außenrohres erreicht werden kann, so dass die Innenröhre nahezu spielfrei in der Außenröhre geführt ist. Hierzu weist die Innenröhre im Schneidbereich - insbesondere im nicht in das Außenrohr eingeführten Zustand - einen Außendurchmesser auf, der zumindest dem Innendurchmesser der Außenröhre entspricht. Das geschlitzte Innenrohr kann beispielsweise nach dem Erzeugen des Schlitzes einer Spreizung im Schneidbereich unterzogen werden oder im Schneidbereich einen größeren Außendurchmesser aufweisen, als im restlichen Bereich - um ein perfektes reproduzierbares paralleles Anliegen von Innenröhre und Außenröhre zu gewährleisten. Insbesondere ist denkbar, dass dies durch mechanisches Aufweiten des Schneidbereichs der Innenröhre - vor dem (ein)Schlitzen - oder durch abhonen des unteren Rohrbereiches realisiert. Dadurch passt sich der Außendurchmesser der Innenröhre an den Innendurchmesser der Außenröhre an und liegt die Innenröhre im Schneidbereich unter Vorspannung in der Außenröhre an. Durch die erfindungsgemäße Konstruktion wird der Schneideffekt verbessert, da sich der Außendurchmesser der Inneröhre dem Innendurchmesser der Außenröhre annähert und somit der Schneideffekt über die gesamte Länge der Schneidkanten läuft und nicht nur eine "linienförmige", reibende Berührung zustande kommt. Zur Montage wird der geschlitzte Bereich der Innenröhre zusammengedrückt und in die Außenröhre eingeführt. Weitere Vorteile der erfindungsgemäßen Vorrichtung bestehen darin, dass die entstehenden Freiflächen den Gegenimpuls der herabfahrenden Innenröhre zur Aspirationsrichtung des aspirierten Gewebes reduzieren, was strömungstechnisch zur geringeren Turbulenzen führt. Die reduzierte Masse ermöglicht weiterhin eine kontrollierte Steuerung bzw. Impulsübertragung auf die Innenschneide mit dem Ziel, die Hubbewegung zu beschleunigen, was zu höheren Schnittrate bzw. einer höheren Impulszahl durch ein Steuergerät führt.

Es ist denkbar, dass die Innenröhre in der Außenröhre alternierend entlang der Längsachse der Vorrichtung verschiebbar ist oder dass die Innenröhre gegenüber der Außenröhre drehbar ist. Die Innenröhre und/oder die Außenröhre können beispielsweise aus Metall, Kunststoff oder aus einem Faserverbundwerkstoff bestehen.

In vorteilhafter Weise kann sich der Schlitz entlang der Längsachse der Innenröhre oder schräg zu der Längsachse der Innenröhre erstrecken. Beliebige Geometrien sind denkbar, wobei wesentlich ist, dass sich die Innenröhre im Schneidbereich zusammendrücken und unter Vorspannung in der Außenröhre führen lässt.

Alternativ oder zusätzlich kann sich der Schlitz in proximaler Richtung über den Schneidbereich hinaus erstrecken.

In besonders vorteilhafter Weise können mehrere Schlitze angeordnet sein. Durch eine solche konstruktive Maßnahme wird erreicht, dass die Innenröhre möglichst symmetrisch unter Vorspannung in der Außenröhre anliegt.

Zur Erhöhung der Schneidleistung sind an der Innenröhre und an der Außenröhre mehrere seitliche Öffnungen mit Schneidkanten vorgesehen. Durch die Schlitzung der Innenröhre wird dabei in idealer Weise gewährleistet, dass die Innenschneidkanten und die Außenschneidkanten optimal aneinander anliegen, was zu einem äußerst guten Schnittergebnis führt. Des Weiteren wird verhindert, dass die Bereiche zwischen den seitlichen Öffnungen beschädigt werden, da sich die Innenröhre aufgrund der Vorspannung nicht schräg zu der Außenröhre bewegt.

Im Konkreten können an der Innenröhre und/oder der Außenröhre zwei seitliche Öffnungen ausgebildet sein, die durch einen Steg voneinander beabstandet sind, wobei sich der Steg schräg zu der Längsachse der Vorrichtung erstreckt. Alternativ können vier seitliche Öffnungen vorgesehen sein, die durch einen kreuzförmigen Steg voneinander getrennt sind.

In weiter erfindungsgemäßer Weise sind die seitlichen Öffnungen wabenförmig, d.h. sechseckig ausgebildet und können in einer Wabenstruktur angeordnet sein.

Die Offenbarung umfasst des Weiteren eine Vorrichtung zum Schneiden und Absaugen von Gewebe aus dem menschlichen oder tierischen Auge, insbesondere zur Durchführung einer Vitrektomie oder eines Netzhautpeelings, mit einer Außenröhre und einer in der Außenröhre bewegbaren Innenröhre, wobei die Außenröhre in einem Schneidbereich wenigstens eine seitliche Öffnung mit zumindest einer Außenschneidkante aufweist, wobei die Innenröhre in dem Schneidbereich mindestens eine seitliche Öffnung mit zumindest einer Innenschneidkante aufweist und wobei die Innenschneidkante und die Außenschneidkante beim Bewegen der Innenröhre gegenüber der Außenröhre schneidend zusammen wirken,
- wobei an der Innenröhre bzw. an der Außenröhre mehrere seitliche Öffnungen vorgesehen sind und wobei die seitlichen Öffnungen wabenförmig ausgebildet bzw. in einer Wabenstruktur angeordnet sind oder
- wobei zwei seitliche Öffnungen ausgebildet sind, die durch einen Steg voneinander beabstandet sind, wobei sich der Steg schräg zur Längsachse der Innenröhre und/oder der Außenröhre erstreckt oder
- wobei an der Innenröhre und/oder an der Außenröhre vier seitliche Öffnungen ausgebildet sind, die durch einen kreuzförmig ausgebildeten Steg voneinander getrennt sind.

Um die Herstellungskosten zu minimieren und eine präzise Ausgestaltung des Schlitzes bzw. der Schlitze zu erreichen, können diese durch Funkenerodieren oder durch Laserschweißen hergestellt sein.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: eine aus dem Stand der Technik bekannte Vorrichtung zum Schneiden und Absaugen von Gewebe aus dem Auge,
- Fig. 2: ein Ausführungsbeispiel einer Vorrichtung zum Schneiden und Absaugen von Gewebe aus dem Auge,
- Fig. 3: ein Ausführungsbeispiel einer Außenröhre einer Vorrichtung zum Schneiden und Absaugen von Gewebe aus dem Auge,
- Fig. 4: ein weiteres Ausführungsbeispiel einer Außenröhre einer Vorrichtung zum Schneiden und Absaugen von Gewebe aus dem Auge,
- Fig. 5: ein weiteres Ausführungsbeispiel einer Außenröhre einer Vorrichtung zum Schneiden und Absaugen von Gewebe aus dem Auge, und
- Fig. 6: in einer schematischen Darstellung ein Ausführungsbeispiel der seitlichen Öffnungen einer Innenröhre und/oder Außenröhre einer erfindungsgemäßen Vorrichtung.

Es wird darauf hingewiesen, dass zur Verbesserung der Übersichtlichkeit in den Figuren teilweise nicht jedes dargestellte Element mit einem Bezugszeichen versehen ist.

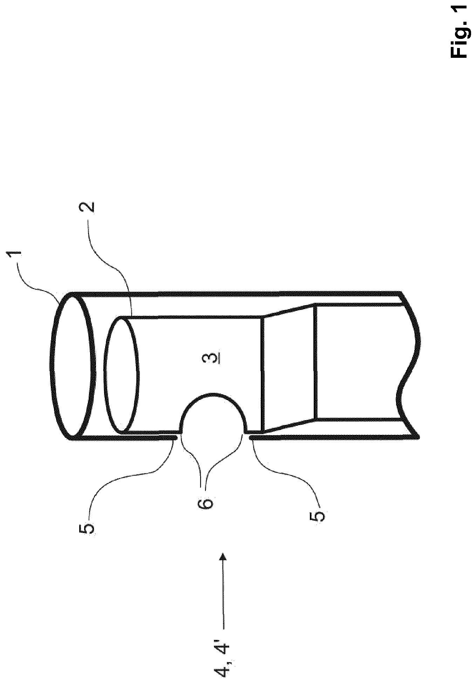

Fig. 1 zeigt den Bereich des distalen Endes einer Vorrichtung zum Schneiden und Absaugen von Gewebe aus dem Auge, insbesondere zum Einsatz in der Vitrektomie. Darin ist deutlich zu erkennen, dass die Vorrichtung eine Außenröhre 1 und eine in der Außenröhre 1 verschiebbar angeordnete Innenröhre 2 aufweist. Die Außenröhre 1 und die Innenröhre 2 umfassen im Schneidbereich 3 jeweils eine seitliche Öffnung 4, 4'. An der seitlichen Öffnung 4 ist zumindest eine Außenschneidkante 5 und an der seitlichen Öffnung 4' ist zumindest eine Innenschneidkante 6 ausgebildet. Die Außenschneidkante 5 und die Innenschneidkante 6 wirken zusammen, nämlich beim Verschieben der Innenröhre 2 gegenüber der Außenröhre 1. Das abgetrennte Gewebe wird mittels Unterdruck durch die Innenröhre 1 aus dem Auge abgesaugt. Um die Innenröhre 1 im Schneidbereich 3 unter Vorspannung in der Außenröhre 1 zu führen, ist die Innenröhre 2 "z-förmig" gebogen. Die Erzeugung der "z-förmigen" Biegung ist Fertigungstechnisch sehr aufwändig und somit kostenintensiv.

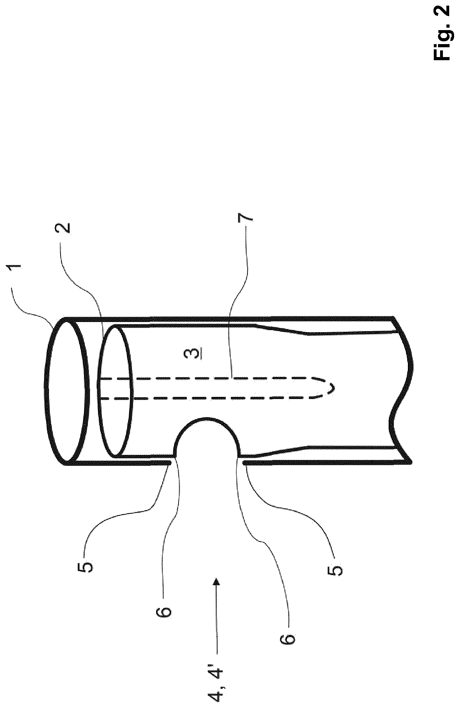

Fig. 2 zeigt den distalen Bereich einer Vorrichtung. Um die Innenröhre 2 im Schneidbereich 3 spielfrei und unter Vorspannung in der Außenröhre 1 zu führen, ist wenigstens ein Schlitz 7 in der Innenröhre 2 ausgebildet. Des Weiteren ist der Außendurchmesser der Innenröhre 2 im Schneidbereich 3, insbesondere wenn die Innenröhre 2 nicht in die Außenröhre 1 eingeführt ist, zumindest so groß wie der Innendurchmesser der Außenröhre 1. Durch den Schlitz 7 kann die Innenröhre 2 im Schneidbereich 3 zusammengedrückt und in die Außenröhre 1 eingeführt werden. Dadurch wird erreicht, dass die Schneidkanten 5, 6 über ihre gesamte Länge hinweg miteinander schneidend zusammenwirken.

Der Schlitz 7 erstreckt sich in dem hier gezeigten Ausführungsbeispiel über den Schneidbereich 3 hinaus und verläuft entlang der Längsachse der Innenröhre 2. Es können jedoch mehrere Schlitze 7 vorgesehen sein. Des Weiteren kann ein einzelner oder mehrere Schlitze 7 schräg zur Längsachse der Innenröhre 2 verlaufen.

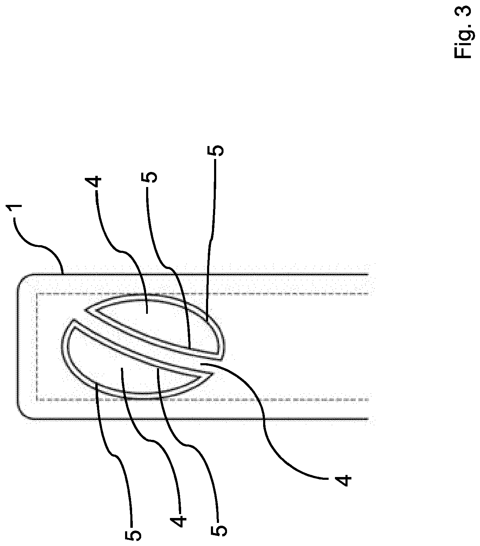

In Fig. 3 ist eine Außenröhre 1 einer Vorrichtung dargestellt. Die Außenröhre 1 weist zwei seitliche Öffnungen 4 mit mehreren Außenschneidkanten 5 auf. Die seitlichen Öffnungen 4 sind durch einen Steg 8 voneinander getrennt, der schräg zur Längsrichtung der Außenröhre 1 verläuft. Des Weiteren ist deutlich zu erkennen, dass die seitlichen Öffnungen 4 halbkreisförmig ausgebildet sind.

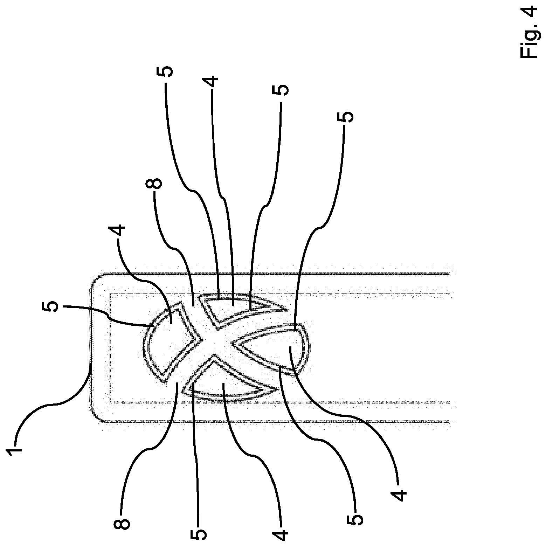

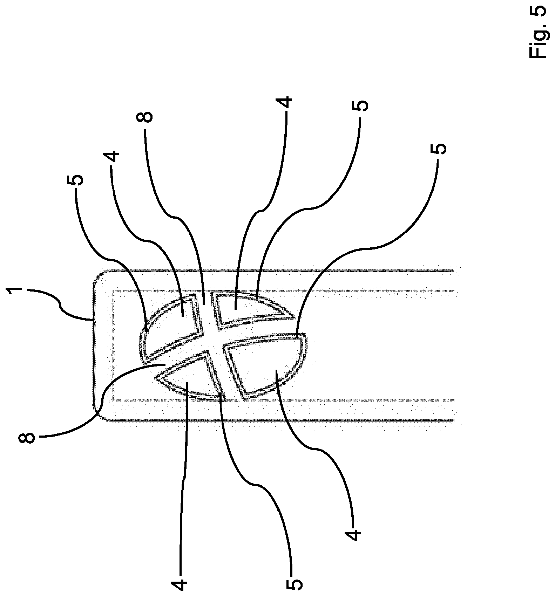

Die Fig. 4 und 5 zeigen weitere Ausführungsbeispiele einer Außenröhre 1 einer Vorrichtung. Dabei sind jeweils vier seitliche Öffnungen 4 mit mehreren Außenschneidkanten 5 realisiert. Bei dieser Ausgestaltung wird die Schneidleistung durch die zusätzlichen Außenschneidkanten 5 nochmals verbessert. Die seitlichen Öffnungen 4 sind durch einen kreuzförmig ausgebildeten Steg 8 voneinander getrennt.

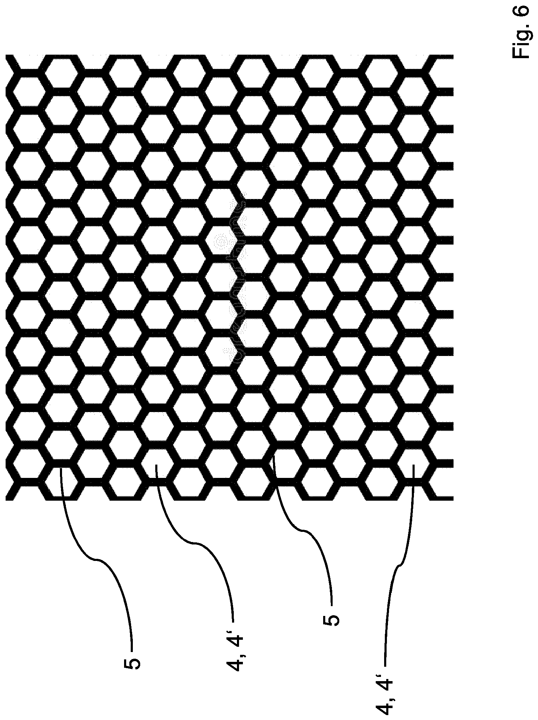

Fig. 6 zeigt in einer schematischen Darstellung ein Ausführungsbeispiel der seitlichen Öffnungen 4, 4' einer Innenröhre 2 und/oder Außenröhre 1 einer erfindungsgemäßen Vorrichtung. Daraus ist deutlich zu erkennen, dass die seitlichen Öffnungen wabenförmig 4, 4' ausgebildet und in einer Wabenstruktur angeordnet sind. Diese Konstruktion zeichnet sich durch eine besondere Stabilität bei geringstmöglichem Materialeinsatz aus.

### Bezugszeichenliste

- 1: Außenröhre
- 2: Innenröhre
- 3: Schneidbereich
- 4, 4': seitliche Öffnung
- 5: Außenschneidkante
- 6: Innenschneidkante
- 7: Schlitz
- 8: Steg

## Patentansprüche

1. Vorrichtung zum Schneiden und Absaugen von Gewebe aus dem menschlichen oder tierischen Auge, insbesondere zur Durchführung einer Vitrektomie oder eines Netzhautpeelings, mit einer Außenröhre (1) und einer in der Außenröhre (1) bewegbaren Innenröhre (2), wobei die Außenröhre (1) in einem Schneidbereich (3) mehrere seitliche Öffnungen (4) mit zumindest jeweils einer Außenschneidkante (5) aufweist, wobei die Innenröhre (2) in dem Schneidbereich (3) mehrere seitliche Öffnungen (4') mit zumindest jeweils einer Innenschneidkante (6) aufweist, wobei die Innenschneidkante (6) und die Außenschneidkante (5) beim Bewegen der Innenröhre (2) gegenüber der Außenröhre (1) schneidend zusammen wirken, wobei in dem Schneidbereich (3) der Außendurchmesser der Innenröhre (2) dem Innendurchmesser der Außenröhre (1) entspricht oder größer als der Innendurchmesser der Außenröhre (1) ist und wobei die Wandung der Innenröhre (2) im Schneidbereich (3) einen Schlitz (7) aufweist,
**dadurch gekennzeichnet, dass** die seitlichen Öffnungen (4) der Außenröhre (1) und/oder die seitlichen Öffnungen (4') der Innenröhre (2) wabenförmig ausgebildet sind.

2. Vorrichtung nach Anspruch 1, **dadurch gekennzeichnet, dass** sich der Schlitz (7) entlang der Längsachse der Innenröhre (2) erstreckt.

3. Vorrichtung nach Anspruch 1, **dadurch gekennzeichnet, dass** sich der Schlitz (7) schräg zu der Längsachse der Innenröhre (2) erstreckt.

4. Vorrichtung nach einem der Ansprüche 1 bis 3, **dadurch gekennzeichnet, dass** sich der Schlitz (7) von dem distalen Ende der Innenröhre (2) über den Schneidbereich (3) hinaus erstreckt.

5. Vorrichtung nach einem der Ansprüche 1 bis 4, **dadurch gekennzeichnet, dass** mehrere Schlitze (7) angeordnet sind.

6. Vorrichtung nach einem der Ansprüche 1 bis 5, **dadurch gekennzeichnet, dass** der/die Schlitz/Schlitze (7) durch Funkenerodieren oder durch Laserschneiden hergestellt ist/sind.

## Claims

1. Apparatus for cutting and drawing off tissue from the human or animal eye, in particular for carrying out a vitrectomy or retina peeling, having an outer tube (1) and an inner tube (2) which can be moved in the outer tube (2), wherein the outer tube (1) in a cutting region (3) has a plurality of lateral openings (4) having at least one outer cutting edge (5) in each case, wherein the inner tube (2) has in the cutting region (3) a plurality of lateral openings (4') having at least one inner cutting edge (6) in each case, wherein the inner cutting edge (6) and the outer cutting edge (5) cooperate in a cutting manner with each other when the inner tube (2) is moved relative to the outer tube (1), wherein in the cutting region (3) the outer diameter of the inner tube (2) corresponds to the inner diameter of the outer tube (1) or is greater than the inner diameter of the outer tube (1) and wherein the wall of the inner tube (2) has a slot (7) in the cutting region (3),
**characterised in that** the lateral openings (4) of the outer tube (1) and/or the lateral openings (4') of the inner tube (2) are constructed in a honeycomb-like manner.

2. Apparatus according to claim 1, **characterised in that** the slot (7) extends along the longitudinal axis of the inner tube (2).

3. Apparatus according to claim 1, **characterised in that** the slot (7) extends obliquely relative to the longitudinal axis of the inner tube (2).

4. Apparatus according to any one of claims 1 to 3, **characterised in that** the slot (7) extends from the distal end of the inner tube (2) beyond the cutting region (3).

5. Apparatus according to any one of claims 1 to 4, **characterised in that** a plurality of slots (7) are arranged.

6. Apparatus according to any one of claims 1 to 5, **characterised in that** the slot(s) (7) is/are produced by means of electrical discharge machining or by means of laser cutting.

## Revendications

1. Dispositif de découpe et d'aspiration d'un tissu de l'oeil humain ou animal, plus particulièrement pour la réalisation d'une vitrectomie ou d'un peeling de la rétine, avec un tuyau externe (1) et un tuyau interne (2) mobile dans le tuyau externe (1), dans lequel le tuyau externe (1) comprend, dans une zone de coupe (3), plusieurs ouvertures latérales (4) avec chacune au moins une arête de coupe externe (5), dans lequel le tuyau interne (2) comprend, dans la zone de coupe (3), plusieurs ouvertures latérales (4') avec chacune au moins une arête de coupe interne (6), dans lequel l'arête de coupe interne (6) et l'arête de coupe externe (5) interagissent en coupant lors du déplacement du tuyau interne (2) par rapport au tuyau externe (1), dans lequel, dans la zone de coupe (3), le diamètre extérieur du tuyau interne (2) correspond au diamètre intérieur du tuyau externe (1) ou est supérieur au diamètre intérieur du tuyau externe (1) et dans lequel la paroi du tuyau interne (2) comprend une fente (7) dans la zone de coupe (3),
**caractérisé en ce que** les ouvertures latérales (4) du tuyau interne (1) et/ou les ouvertures latérales (4') du tuyau interne (2) présentent des formes d'alvéoles.

2. Dispositif selon la revendication 1, **caractérisé en ce que** la fente (7) s'étend le long de l'axe longitudinal du tuyau interne (2).

3. Dispositif selon la revendication 1, **caractérisé en ce que** la fente (7) s'étend de manière oblique par rapport à l'axe longitudinal du tuyau interne (2).

4. Dispositif selon l'une des revendications 1 à 3, **caractérisé en ce que** la fente (7) s'étend à partir de l'extrémité distale du tuyau interne (2) au-delà de la zone de coupe (3).

5. Dispositif selon l'une des revendications 1 à 4, **caractérisé en ce que** plusieurs fentes (7) sont présentes.

6. Dispositif selon l'une des revendications 1 à 5, **caractérisé en ce que** la ou les fentes (7) est ou sont réalisée(s) par électro-érosion ou par découpe au laser.
